# EUROPEAN PATENT APPLICATION

(11) **EP 3 711 571 A1**
(43) Date of publication of application: **23.09.2020**
(21) Application number: 19020207.7
(22) Date of filing: 22.03.2019
(51) Int. Cl.: A24F 47/00, A61M 15/06, A61M 11/04

(54) **SMOKING SUBSTITUTE SYSTEM WITH EXTERNAL PANEL TO PROVIDE THERMAL INSULATION TO AN INTERNAL HOUSING**

(71) Applicant: NERUDIA LIMITED, Liverpool Merseyside L24 9HP (GB)
(72) Inventor: Ferrie, Kate, Liverpool L24 9HP (GB); Shenton, Ross, Liverpool L24 9HP (GB); Lord, Chris, Liverpool L24 9HP (GB); Murray, Samantha, Liverpool L24 9HP (GB)
(74) Representative: Mewburn Ellis LLP

(57) **Abstract**

A smoking substitute device comprising: a housing configured to house components of the smoking substitute device; and a panel configured to cover an external surface of the housing; wherein the panel is configured to space from said external surface so as to provide thermal insulation to the housing.

## Description

### TECHNICAL FIELD

The present invention relates to a smoking substitute system and particularly, although not exclusively, to a smoking substitute system comprising a device and an aerosol-forming article.

### BACKGROUND

The smoking of tobacco is generally considered to expose a smoker to potentially harmful substances. It is generally thought that a significant amount of the potentially harmful substances are generated through the heat caused by the burning and/or combustion of the tobacco and the constituents of the burnt tobacco in the tobacco smoke itself.

Conventional combustible smoking articles, such as cigarettes, typically comprise a cylindrical rod of tobacco comprising shreds of tobacco which is surrounded by a wrapper, and usually also a cylindrical filter axially aligned in an abutting relationship with the wrapped tobacco rod. The filter typically comprises a filtration material which is circumscribed by a plug wrap. The wrapped tobacco rod and the filter are joined together by a wrapped band of tipping paper that circumscribes the entire length of the filter and an adjacent portion of the wrapped tobacco rod. A conventional cigarette of this type is used by lighting the end opposite to the filter, and burning the tobacco rod. The smoker receives mainstream smoke into their mouth by drawing on the mouth end or filter end of the cigarette.

Combustion of organic material such as tobacco is known to produce tar and other potentially harmful by-products. There have been proposed various smoking substitute systems (or "substitute smoking systems") in order to avoid the smoking of tobacco.

Such smoking substitute systems can form part of nicotine replacement therapies aimed at people who wish to stop smoking and overcome a dependence on nicotine.

Smoking substitute systems include electronic systems that permit a user to simulate the act of smoking by producing an aerosol (also referred to as a "vapour") that is drawn into the lungs through the mouth (inhaled) and then exhaled. The inhaled aerosol typically bears nicotine and/or flavourings without, or with fewer of, the odour and health risks associated with traditional smoking.

In general, smoking substitute systems are intended to provide a substitute for the rituals of smoking, whilst providing the user with a similar experience and satisfaction to those experienced with traditional smoking and with combustible tobacco products. Some smoking substitute systems use smoking substitute articles (also referred to as a "consumables") that are designed to resemble a traditional cigarette and are cylindrical in form with a mouthpiece at one end.

The popularity and use of smoking substitute systems has grown rapidly in the past few years. Although originally marketed as an aid to assist habitual smokers wishing to quit tobacco smoking, consumers are increasingly viewing smoking substitute systems as desirable lifestyle accessories.

There are a number of different categories of smoking substitute systems, each utilising a different smoking substitute approach.

One approach for a smoking substitute system is the so-called Heated Tobacco ("HT") approach in which tobacco (rather than an "e-liquid") is heated or warmed to release vapour. HT is also known as "heat not burn" ("HNB"). The tobacco may be leaf tobacco or reconstituted tobacco. The vapour may contain nicotine and/or flavourings. In the HT approach the intention is that the tobacco is heated but not burned, i.e. the tobacco does not undergo combustion.

A typical HT smoking substitute system may include a device and a consumable. The consumable may include the tobacco material. The device and consumable may be configured to be physically coupled together. In use, heat may be imparted to the tobacco material by a heating element of the device, wherein airflow through the tobacco material causes components in the tobacco material to be released as vapour. A vapour may also be formed from a carrier in the tobacco material (this carrier may for example include propylene glycol and/or vegetable glycerine) and additionally volatile compounds released from the tobacco. The released vapour may be entrained in the airflow drawn through the tobacco.

As the vapour passes through the consumable (entrained in the airflow) from the location of vaporisation to an outlet of the consumable (e.g. a mouthpiece), the vapour cools and condenses to form an aerosol for inhalation by the user. The aerosol will normally contain the volatile compounds.

In HT smoking substitute systems, heating as opposed to burning the tobacco material is believed to cause fewer, or smaller quantities, of the more harmful compounds ordinarily produced during smoking. Consequently, the HT approach may reduce the odour and/or health risks that can arise through the burning, combustion and pyrolytic degradation of tobacco.

In operation, commonly available smoking substitute systems often operate at elevated temperatures. In particular, HT smoking substitute devices may operate at temperatures up to several hundred degree Celsius. Even they are well insulated, the heat generated at the heating element may slowly conduct through the devices' housing to their external surfaces during prolong use. Not only the devices are made difficult to hold, it may also present a hazard.

There may be a need for improved design of smoking substitute systems, in particular HT smoking substitute systems, to enhance the user experience and improve the function of the HT smoking substitute system.

The present disclosure has been devised in the light of the above considerations.

### SUMMARY OF THE INVENTION

At its most general, the present invention relates to improved thermal insulation in a smoking substitute device by providing an air gap at the surface of the device. The air gap may provide additional thermal insulation such that a user may be shielded from the heat resulting from prolong use of the device.

According to a first aspect of the present invention, there is provided a smoking substitute device comprising a housing configured to house components of the smoking substitute device, and a panel configured to cover an external surface of the housing. The panel is configured to space from said external surface so as to provide thermal insulation to the housing.

More specifically, an air gap may be formed between the panel and the housing. The external surface may be one or more major surfaces of the housing where the user grips onto when using the device, for example the front and back surfaces of the housing.

By providing an air gap between a panel and the housing of the smoking substitute device, thermal insulation may be provided for a user during the use of the device. Advantageously, this may improve the overall user experience, in particular during prolong use of the device. Further, such arrangement may reduce the risk of heat related injuries to the user.

Optional features will now be set out. These are applicable singly or in any combination with any aspect.

Optionally, the panel is removably attachable to the housing. For example, damaged panels may be replaced and therefore advantageously such arrangement prolongs the useful life of the device. Moreover, the panel may be interchangeable with panels of another design, as such that the users may adapt a different panel according to their needs. For example, said panels may be formed form different materials such as metal, ceramic, elastomer, wood and other suitable materials. The panels may vary in thickness to provide different levels of thermal insulation. The panels may have different surface finishes, such as a smooth finish or a tactile finish. The panel may have different forms of protrusion extending from its outer surface to provide additional grip and thermal insulation, for example dots, grooves and protrusions of any pattern.

Optionally, the panel is configured to allow access to a user interface positioned on the external surface of the housing. For example, a portion of the panel adjacent to the user interface may be deformable such that the user may interact with the user interface through said deformable portion. Alternatively, an aperture may be formed on the panel adjacent to the user interface such that the user may interact with the user interface through said aperture. Advantageously, such arrangement may conceal the user interface, as well as forming a protective shield for the interface.

Optionally, the panel comprises a window adjacent to a visual indicator provided on the housing. The window may be an aperture formed on the panel, or it may be a transparent or translucent portion formed on the panel. Advantageously, such arrangement may allow output, e.g. light signal, from the visual indicator to be observed through the panel.

Optionally, the panel comprises a first panel configured to cover a first external surface at the front of the device and a second panel configured to cover a second external surface at the back of the device opposite to the front of the device. Advantageously, air gaps are provided to both the front and back of the device and thereby it may improve the overall thermal insulation of the device.

Optionally, the first panel is configured to engage with the second panel when the first panel and the second panel cover the respective external surface of the housing. For example, the first panel and the second panel may form a shell or sleeve for enclosing the device. Advantageously, this may further improve the overall thermal insulation of the device because the sides of the housing may also be covered by the first panel and second panel when they are engaged with each other.

Optionally, the first panel is configured to form a snap lock engagement with the second panel. Optionally, the panel is configured to cover the external surface by attaching to the housing with one or more of a magnetic engagement, a living hinge engagement, a snap groove engagement, or runner formed between the panel and the housing. Advantageously, such arrangement allows the panels to be replaced more readily, without the use of a tool.

Optionally, one or more vents are defined between the external surface and the panel. For example, the one or more vents may be perforations formed on the panel, or grooves formed between the panel and the devices. Adventurously, such arrangement may aid the cooling of the device and may also prevent any pressure build up in the gap due to heat cycles. Advantageously, the grooves may further improve thermal insulation of the device, as well as improving the strength of the panel such that it may be less likely to deform.

Optionally, the panel comprises a non-scratch surface finish. Optionally, the panel comprises an elastic coating. Advantageously, such arrangements reduces the likelihood of scratch or damage to the external surface of the housing.

Optionally, a portion of the panel comprises a planar surface corresponding to the external surface of the device. For example, the planar surface corresponds to a portion of planar external surface of the device.

Optionally, the smoking substitute device is a heat-not-burn (HNB) device.

Optionally, the panel is configured to be visible from only one side of the device.

The device may comprise an elongate body. An end of the elongate body may be configured for engagement with an aerosol-forming article. For example, the body may be configured for engagement with a heated tobacco (HT) consumable (or heat-not-burn (HNB) consumable). The terms "heated tobacco" and "heat-not-burn" are used interchangeably herein to describe a consumable that is of the type that is heated rather than combusted (or are used interchangeably to describe a device for use with such a consumable). The device may comprise a cavity that is configured for receipt of at least a portion of the consumable (i.e. for engagement with the consumable). The aerosol-forming article may be of the type that comprises an aerosol former (e.g. carried by an aerosol-forming substrate).

The device may comprise a heater for heating the aerosol-forming article. The heater may comprise a heating element, which may be in the form of a rod that extends from the body of the device. The heating element may extend from the end of the body that is configured for engagement with the aerosol-forming article.

The heater (and thus the heating element) may be rigidly mounted to the body. The heating element may be elongate so as to define a longitudinal axis and may, for example, have a transverse profile (i.e. transverse to a longitudinal axis of the heating element) that is substantially circular (i.e. the heating element may be generally cylindrical). Alternatively, the heating element may have a transverse profile that is rectangular (i.e. the heater may be a "blade heater"). The heating element may alternatively be in the shape of a tube (i.e. the heater may be a "tube heater"). The heating element may take other forms (e.g. the heating element may have an elliptical transverse profile). The shape and/or size (e.g. diameter) of the transverse profile of the heating element may be generally consistent for the entire length (or substantially the entire length) of the heating element.

The heating element may be between 15 mm and 25 mm long, e.g. between 18 mm and 20 mm long, e.g. around 19 mm long. The heating element may have a diameter of between 1.5 mm and 2.5 mm, e.g. a diameter between 2 mm and 2.3 mm, e.g. a diameter of around 2.15 mm.

The heating element may be formed of ceramic. The heating element may comprise a core (e.g. a ceramic core) comprising Al2O3. The core of the heating element may have a diameter of 1.8 mm to 2.1 mm, e.g. between 1.9 mm and 2 mm. The heating element may comprise an outer layer (e.g. an outer ceramic layer) comprising Al2O3. The thickness of the outer layer may be between 160 µm and 220 µm, e.g. between 170 µm and 190 µm, e.g. around 180 µm. The heating element may comprise a heating track, which may extend longitudinally along the heating element. The heating track may be sandwiched between the outer layer and the core of the heating element. The heating track may comprise tungsten and/or rhenium. The heating track may have a thickness of around 20 µm.

The heating element may be located in the cavity (of the device), and may extend (e.g. along a longitudinal axis) from an internal base of the cavity towards an opening of the cavity. The length of the heating element (i.e. along the longitudinal axis of the heater) may be less than the depth of the cavity. Hence, the heating element may extend for only a portion of the length of the cavity. That is, the heating element may not extend through (or beyond) the opening of the cavity.

The heating element may be configured for insertion into an aerosol-forming article (e.g. a HT consumable) when an aerosol-forming article is received in the cavity. In that respect, a distal end (i.e. distal from a base of the heating element where it is mounted to the device) of the heating element may comprise a tapered portion, which may facilitate insertion of the heating element into the aerosol-forming article. The heating element may fully penetrate an aerosol-forming article when the aerosol-forming article is received in the cavity. That is, the entire length, or substantially the entire length, of the heating element may be received in the aerosol-forming article.

The heating element may have a length that is less than, or substantially the same as, an axial length of an aerosol-forming substrate forming part of an aerosol-forming article (e.g. a HT consumable). Thus, when such an aerosol-forming article is engaged with the device, the heating element may only penetrate the aerosol-forming substrate, rather than other components of the aerosol-forming article. The heating element may penetrate the aerosol-forming substrate for substantially the entire axial length of the aerosol forming-substrate of the aerosol-forming article. Thus, heat may be transferred from (e.g. an outer circumferential surface of) the heating element to the surrounding aerosol-forming substrate, when penetrated by the heating element. That is, heat may be transferred radially outwardly (in the case of a cylindrical heating element) or e.g. radially inwardly (in the case of a tube heater).

Where the heater is a tube heater, the heating element of the tube heater may surround at least a portion of the cavity. When the portion of the aerosol-forming article is received in the cavity, the heating element may surround a portion of the aerosol-forming article (i.e. so as to heat that portion of the aerosol-forming article). In particular, the heating element may surround an aerosol forming substrate of the aerosol-forming article. That is, when an aerosol-forming article is engaged with the device, the aerosol forming substrate of the aerosol-forming article may be located adjacent an inner surface of the (tubular) heating element. When the heating element is activated, heat may be transferred radially inwardly from the inner surface of the heating element to heat the aerosol forming substrate.

The cavity may comprise a (e.g. circumferential) wall (or walls) and the (tubular) heating element may extend around at least a portion of the wall(s). In this way, the wall may be located between the inner surface of the heating element and an outer surface of the aerosol-forming article. The wall (or walls) of the cavity may be formed from a thermally conductive material (e.g. a metal) to allow heat conduction from the heating element to the aerosol-forming article. Thus, heat may be conducted from the heating element, through the cavity wall (or walls), to the aerosol-forming substrate of an aerosol-forming article received in the cavity.

In some embodiments the heater may form part of an aerosol-forming article for use with the device. In such cases the device may not comprise a heater. Rather, the aerosol-forming article may comprise a heater. Such arrangements may, for example, be suited to e-cigarette systems in which the aerosol-forming article comprises a tank containing an aerosol former (e.g. in liquid form). In such embodiments, the device may comprise means for connecting the device the heater of an aerosol-forming article engaged with the device. For example, the device may comprise one or more device connectors for (e.g. electrically) connecting the device to a corresponding heater connector of the aerosol-forming article. The connectors (i.e. of both the device and the aerosol-forming article) may be in the form of electrically conductive elements (e.g. plates) that contact when the aerosol-forming article is engaged with the device.

In some embodiments the device may comprise a cap disposed at the end of the body that is configured for engagement with an aerosol-forming article. Where the device comprises a heater having a heating element, the cap may at least partially enclose the heating element. The cap may be moveable between an open position in which access is provided to the heating element, and a closed position in which the cap at least partially encloses the heating element. The cap may be slideably engaged with the body of the device, and may be slideable between the open and closed positions.

The cap may define at least a portion of the cavity of the device. That is, the cavity may be fully defined by the cap, or each of the cap and body may define a portion of the cavity. Where the cap fully defines the cavity, the cap may comprise an aperture for receipt of the heating element into the cavity (when the cap is in the closed position). The cap may comprise an opening to the cavity. The opening may be configured for receipt of at least a portion of an aerosol-forming article. That is, an aerosol-forming article may be inserted through the opening and into the cavity (so as to be engaged with the device).

The cap may be configured such that when an aerosol-forming article is engaged with the device (e.g. received in the cavity), only a portion of the aerosol-forming article is received in the cavity. That is, a portion of the aerosol-forming article (not received in the cavity) may protrude from (i.e. extend beyond) the opening. This (protruding) portion of the aerosol-forming article may be a terminal (e.g. mouth) end of the aerosol-forming article, which may be received in a user's mouth for the purpose of inhaling aerosol formed by the device.

The device may comprise a power source or may be connectable to a power source (e.g. a power source separate to the device). The power source may be electrically connectable to the heater. In that respect, altering (e.g. toggling) the electrical connection of the power source to the heater may affect a state of the heater. For example, toggling the electrical connection of the power source to the heater may toggle the heater between an on state and an off state. The power source may be a power store. For example, the power source may be a battery or rechargeable battery (e.g. a lithium ion battery).

The device may comprise an input connection (e.g. a USB port, Micro USB port, USB-C port, etc.). The input connection may be configured for connection to an external source of electrical power, such as a mains electrical supply outlet. The input connection may, in some cases, be used as a substitute for an internal power source (e.g. battery or rechargeable battery). That is, the input connection may be electrically connectable to the heater (for providing power to the heater). Hence, in some forms, the input connection may form at least part of the power source of the device.

Where the power source comprises a rechargeable power source (such as a rechargeable battery), the input connection may be used to charge and recharge the power source.

The device may comprise a user interface (Ul). In some embodiments the UI may include input means to receive operative commands from the user. The input means of the UI may allow the user to control at least one aspect of the operation of the device. In some embodiments the input means may comprise a power button to switch the device between an on state and an off state.

In some embodiments the UI may additionally or alternatively comprise output means to convey information to the user. In some embodiments the output means may comprise a light to indicate a condition of the device (and/or the aerosol-forming article) to the user. The condition of the device (and/or aerosol-forming article) indicated to the user may comprise a condition indicative of the operation of the heater. For example, the condition may comprise whether the heater is in an off state or an on state. In some embodiments, the UI unit may comprise at least one of a button, a display, a touchscreen, a switch, a light, and the like. For example, the output means may comprise one or more (e.g. two, three, four, etc.) light-emitting diodes ("LEDs") that may be located on the body of the device.

The device may further comprise a puff sensor (e.g. airflow sensor), which form part of the input means of the Ul. The puff sensor may be configured to detect a user drawing on an end (i.e. a terminal (mouth) end) of the aerosol-forming article. The puff sensor may, for example, be a pressure sensor or a microphone. The puff sensor may be configured to produce a signal indicative of a puff state. The signal may be indicative of the user drawing (an aerosol from the aerosol-forming article) such that it is e.g. in the form of a binary signal. Alternatively or additionally, the signal may be indicative of a characteristic of the draw (e.g. a flow rate of the draw, length of time of the draw, etc.).

The device may comprise a controller, or may be connectable to a controller that may be configured to control at least one function of the device. The controller may comprise a microcontroller that may e.g. be mounted on a printed circuit board (PCB). The controller may also comprise a memory, e.g. non-volatile memory. The memory may include instructions, which, when implemented, may cause the controller to perform certain tasks or steps of a method. Where the device comprises an input connection, the controller may be connected to the input connection.

The controller may be configured to control the operation of the heater (and e.g. the heating element). Thus, the controller may be configured to control vaporisation of an aerosol forming part of an aerosol-forming article engaged with the device. The controller may be configured to control the voltage applied by power source to the heater. For example, the controller may be configured to toggle between applying a full output voltage (of the power source) to the heater and applying no voltage to the heater. Alternatively or additionally, the control unit may implement a more complex heater control protocol.

The device may further comprise a voltage regulator to regulate the output voltage supplied by the power source to form a regulated voltage. The regulated voltage may subsequently be applied to the heater.

In some embodiments, where the device comprises a Ul, the controller may be operatively connected to one or more components of the UI. The controller may be configured to receive command signals from an input means of the UI. The controller may be configured to control the heater in response to the command signals. For example, the controller may be configured to receive "on" and "off command signals from the UI and, in response, may control the heater so as to be in a corresponding on or off state.

The controller may be configured to send output signals to a component of the UI. The UI may be configured to convey information to a user, via an output means, in response to such output signals (received from the controller). For example, where the device comprises one or more LEDs, the LEDs may be operatively connected to the controller. Hence, the controller may configured to control the illumination of the LEDs (e.g. in response to an output signal). For example, the controller may be configured to control the illumination of the LEDs according to (e.g. an on or off) state of the heater.

Where the device comprises a sensor (e.g. a puff/airflow sensor), the controller may be operatively connected to the sensor. The controller may be configured to receive a signal from the sensor (e.g. indicative of a condition of the device and/or engaged aerosol-forming article). The controller may be configured to control the heater, or an aspect of the output means, based on the signal from the sensor.

The device may comprise a wireless interface configured to communicate wirelessly (e.g. via Bluetooth (e.g. a Bluetooth low-energy connection) or WiFi) with an external device. Similarly, the input connection may be configured for wired connection to an external device so as to provide communication between the device and the external device.

The external device may be a mobile device. For example, the external device may be a smart phone, tablet, smart watch, or smart car. An application (e.g. app) may be installed on the external device (e.g. mobile device). The application may facilitate communication between the device and the external device via the wired or wireless connection.

The wireless or wired interface may be configured to transfer signals between the external device and the controller of the device. In this respect, the controller may control an aspect of the device in response to a signal received from an external device. Alternatively or additionally, an external device may respond to a signal received from the device (e.g. from the controller of the device).

According to a second aspect of the present invention, there is provided a smoking substitute system comprising a smoking substitute device according to the first aspect and an aerosol-forming article. The aerosol-forming article may comprise an aerosol-forming substrate at an upstream end of the aerosol-forming article. Optionally, the article may be in the form of a smoking substitute article, e.g. heated tobacco (HT) consumable (also known as a heat-not-burn (HNB) consumable).

As used herein, the terms "upstream" and "downstream" are intended to refer to the flow direction of the vapour/aerosol i.e. with the downstream end of the article/consumable being the mouth end or outlet where the aerosol exits the consumable for inhalation by the user. The upstream end of the article/consumable is the opposing end to the downstream end.

The aerosol-forming substrate is capable of being heated to release at least one volatile compound that can form an aerosol. The aerosol-forming substrate may be located at the upstream end of the article/consumable.

In order to generate an aerosol, the aerosol-forming substrate comprises at least one volatile compound that is intended to be vaporised/aerosolised and that may provide the user with a recreational and/or medicinal effect when inhaled. Suitable chemical and/or physiologically active volatile compounds include the group consisting of: nicotine, cocaine, caffeine, opiates and opoids, cathine and cathinone, kavalactones, mysticin, beta-carboline alkaloids, salvinorin A together with any combinations, functional equivalents to, and/or synthetic alternatives of the foregoing.

The aerosol-forming substrate may comprise plant material. The plant material may comprise least one plant material selected from the list including *Amaranthus dubius, Arctostaphylos uva-ursi* (Bearberry), *Argemone mexicana, Amica, Artemisia vulgaris,* Yellow Tees, *Galea zacatechichi, Canavalia maritima* (Baybean), *Cecropia mexicana* (Guamura), *Cestrum noctumum, Cynoglossum virginianum* (wild comfrey), *Cytisus scoparius, Damiana, Entada rheedii, Eschscholzia califomica* (California Poppy), *Fittonia albivenis, Hippobroma longiflora, Humulus japonica* (Japanese Hops), *Humulus lupulus* (Hops), *Lactuca virosa* (Lettuce Opium), *Laggera alata, Leonotis leonurus, Leonurus cardiaca* (Motherwort), *Leonurus sibiricus* (Honeyweed), *Lobelia cardinalis, Lobelia inflata* (Indian-tobacco), *Lobelia siphilitica, Nepeta cataria* (Catnip), *Nicotiana species* (Tobacco), *Nymphaea alba* (White Lily), *Nymphaea caerulea* (Blue Lily), Opium poppy, *Passiflora incamata* (Passionflower), *Pedicularis densiflora* (Indian Warrior), *Pedicularis groenlandica* (Elephant's Head), *Salvia divinorum, Salvia dorrii* (Tobacco Sage), Salvia species (Sage), *Scutellaria galericulata, Scutellaria lateriflora, Scutellaria nana, Scutellaria* species (Skullcap), *Sida acuta* (Wireweed), *Sida rhombifolia, Silene capensis, Syzygium aromaticum* (Clove), *Tagetes lucida* (Mexican Tarragon), *Tarchonanthus camphoratus, Tumera diffusa* (Damiana), *Verbascum* (Mullein), *Zamia latifolia* (Maconha Brava) together with any combinations, functional equivalents to, and/or synthetic alternatives of the foregoing.

The plant material may be tobacco. Any type of tobacco may be used. This includes, but is not limited to, flue-cured tobacco, burley tobacco, Maryland Tobacco, dark-air cured tobacco, oriental tobacco, dark-fired tobacco, perique tobacco and rustica tobacco. This also includes blends of the above mentioned tobaccos.

The tobacco may comprise one or more of leaf tobacco, stem tobacco, tobacco powder, tobacco dust, tobacco derivatives, expanded tobacco, homogenised tobacco, shredded tobacco, extruded tobacco, cut rag tobacco and/or reconstituted tobacco (e.g. slurry recon or paper recon).

The aerosol-forming substrate may comprise a gathered sheet of homogenised (e.g. paper/slurry recon) tobacco or gathered shreds/strips formed from such a sheet.

The aerosol-forming substrate may comprise one or more additives selected from humectants, flavourants, fillers, aqueous/non-aqueous solvents and binders.

The flavourant may be provided in solid or liquid form. It may include menthol, liquorice, chocolate, fruit flavour (including e.g. citrus, cherry etc.), vanilla, spice (e.g. ginger, cinnamon) and tobacco flavour. The flavourant may be evenly dispersed throughout the aerosol-forming substrate or may be provided in isolated locations and/or varying concentrations throughout the aerosol-forming substrate.

The aerosol-forming substrate may be formed in a substantially cylindrical shape such that the article/consumable resembles a conventional cigarette. It may have a diameter of between 5 and 10mm e.g. between 6 and 9mm or 6 and 8mm e.g. around 7 mm. It may have an axial length of between 10 and 15mm e.g. between 11 and 14mm such as around 12 or 13mm.

The article/consumable may comprise at least one filter element. There may be a terminal filter element at the downstream/mouth end of the article/consumable.

The or at least one of the filter element(s) (e.g. the terminal filter element) may be comprised of cellulose acetate or polypropylene tow. The at least one filter element (e.g. the terminal filter element) may be comprised of activated charcoal. The at least one filter element (e.g. the terminal element) may be comprised of paper. The or each filter element may be at least partly (e.g. entirely) circumscribed with a plug wrap e.g. a paper plug wrap.

The terminal filter element (at the downstream end of the article/consumable) may be joined to the upstream elements forming the article/consumable by a circumscribing tipping layer e.g. a tipping paper layer. The tipping paper may have an axial length longer than the axial length of the terminal filter element such that the tipping paper completely circumscribes the terminal filter element plus the wrapping layer surrounding any adjacent upstream element.

In some embodiments, the article/consumable may comprise an aerosol-cooling element which is adapted to cool the aerosol generated from the aerosol-forming substrate (by heat exchange) before being inhaled by the user.

The article/consumable may comprise a spacer element that defines a space or cavity between the aerosol-forming substrate and the downstream end of the consumable. The spacer element may comprise a cardboard tube. The spacer element may be circumscribed by the (paper) wrapping layer.

In some embodiments the system may be in the form of an e-cigarette system (i.e. rather than a heated tobacco system as described above). In such a system, the consumable may be in the form of an e-cigarette consumable. The e-cigarette system may be configured such that the consumable can be received and retained in the cavity of the device (i.e. so as to be engaged with the device). The consumable may be retained by way of e.g. an interference fit, screwing one onto (or onto) the other, a bayonet fitting, or by way of a snap engagement mechanism.

The consumable may comprise a tank, which may define a reservoir for the storage of an aerosol former. The aerosol former may be in the form of an e-liquid (stored in the reservoir).

The consumable may be a "single-use" consumable. That is, upon exhausting the e-liquid in the tank, the intention may be that the user disposes of the entire consumable. Alternatively, the e-liquid may be the only part of the system that is truly "single-use". For example, the tank may be refillable with e-liquid or another component of the system (internal to the device or external to the device e.g. a refillable cartomizer) may define a reservoir for the e-liquid.

As set forth above, the consumable may comprise a heater (i.e. instead of the heater forming part of the device) configured to heat and vaporise the e-liquid. The consumable may comprise a porous wick that conveys e-liquid from the tank to a heating element of the heater. The heating element may be a heating filament that is wound (e.g. helically) around at least a portion of the porous wick, such that when the heating element is heated (e.g. by the action of electrical current passing through the heating element), heat may be transferred from the heating element to the e-liquid conveyed by the wick. This transfer of heat may vaporise the e-liquid and the resultant vapour may be entrained in an airflow passing through the consumable.

The consumable may further comprise one or more heater connectors for connecting the heater (of the consumable) to the device. The heater connectors may be in the form of electrically conductive element or contacts (e.g. metal plates) and may be disposed on an in-use device-facing surface of the consumable. The heater connectors may be electrically connected to the heater of the consumable, such that electricity supplied via the heater connectors may pass to the heater. In other words, a voltage applied across the heater connectors may generally correspond to a voltage applied across the heating element of the heater.

The heater connectors may be arranged such that they contact corresponding device connectors of the device when the consumable is engaged with the device. The device connectors may be connected (e.g. electrically) to a power source (e.g. battery) of the device. Thus, electricity may be supplied from the power source to the heating element, via in-contact heater and device connectors. In this way, the heater forming part of the consumable may operate (and interact with e.g. a controller) as otherwise described above with respect to a heater forming part of the device.

According to a third aspect of the present invention, there is provided a method of using the system according to the second aspect, the method comprising inserting the aerosol-forming article into the device; and heating the article using the heater of the device.

In some embodiments the method may comprise inserting the article into a cavity within a body of the device and penetrating the article with the heating element of the device upon insertion of the article.

Present disclosure provides for heat insulation for a smoking substitute device. The device may have the housing and at least one panel. The housing may be configured for housing components of the device such as, the heater, the power source, the controller, the puff sensor, and like. The housing may define an external surface. The user interface may be positioned on the external surface of the housing. For example, the button and the LEDs may be arranged on the external surface.

The panel may be configured for covering at least a portion of the external surface of the housing, The panel may be further configured to space from the external surface to define a gap between an internal surface of the panel and the external surface of the housing. The gap may be filled with air. The panel may have shape and configuration as suitable according to the shape and size of the housing and/or the device. The panel may include a first panel configured for placement over the external surface at a front portion of the housing and a second panel configured for placement over the external surface on a back portion of the housing.

The panel may be configured as required based on the configuration of the housing. The first panel may be provided with a cut-out corresponding to the shape and position of the UI of the device. The cut-out may make the UI accessible to a user. Any other suitable arrangement may be used. For example, in an embodiment, instead of or in addition to the cut-out, a transparent covering may be provided such that UI may be accessible to a user.

The gap may be configured based on insulation requirement. For example, the gap may be provided either over substantially entire length of the housing or it may be localized based on location of the heat generation elements in the housing. Similarly, the shape and structure of the gap may be provided as required. The gap may be more at the front portion and the back portion, and lesser at the side portions of the device.

Further, the panel may be removably attached with the device using a suitable means. Preferably, the panel may be configured for a snap engagement with the device. The snap engagement may provide for an easy mounting and/or unmounting of the panel from the device. In addition, the panel may be provided with different designs, colours, finishes or material. This may enable the users to customize the appearance and feel of the device according to their personal preferences. For example, the panel may be provided with a non-scratch surface finish to prevent formation of any scratches on the panel. In an embodiment, the panel may be provided with a coating of a deformable material such as rubber or silicon to enhance grip of the device or to improve shock resistance of the device. The panel may be made from a breakable or a non-breakable material as required.

The first panel may be configured for snap engagement with the second panel for retainment of the panel over the external surface. Longitudinal edges of the first panel and the second panel may be configured for removable attachment with each other. The longitudinal edge of the first panel snap may lock with the longitudinal edge of the second panel for mounting the panel over and covering the external surface of the housing. Further, the first panel may be provided with a rib defining the periphery of the cut-out. The rib may be configured for positioning inside a groove provided in the housing surrounding the UI. For mounting the first panel on the housing, the first panel may either be pushed into a snap lock engagement of the rib in the groove, or the first panel may be configured to be slid along a longitudinal axis of the housing for inserting the rib in the groove. Any suitable provision may be provided as required.

In an embodiment, the panel may be provided with at least one perforation. The perforations may provide for passage of air between the gap and the environment. The passage of air may provide for a venting of heated air from the gap and for receiving cool air from the environment. This may aid in cooling of the device and may also prevent any pressure build up in the gap due to repeated heating/cooling of the device. The perforations may be in form of holes in the panel or may be in form of crevices between the housing and the panel.

The invention includes the combination of the aspects and preferred features described except where such a combination is clearly impermissible or expressly avoided.

The skilled person will appreciate that except where mutually exclusive, a feature or parameter described in relation to any one of the above aspects may be applied to any other aspect. Furthermore, except where mutually exclusive, any feature or parameter described herein may be applied to any aspect and/or combined with any other feature or parameter described herein.

### SUMMARY OF THE FIGURES

So that the invention may be understood, and so that further aspects and features thereof may be appreciated, embodiments illustrating the principles of the invention will now be discussed in further detail with reference to the accompanying figures, in which:
Figure 1A is a schematic of a smoking substitute system;
Figure 1B is a schematic of a variation of the smoking substitute system of Figure 1A;
Figure 2A is a front view of a first embodiment of a smoking substitute system with the consumable engaged with the device;
Figure 2B is a front view of the first embodiment of the smoking substitute system with the consumable disengaged from the device;
Figure 2C is a section view of the consumable of the first embodiment of the smoking substitute system;
Figure 2D is a detailed view of an end of the device of the first embodiment of the smoking substitute system;
Figure 2E is a section view of the first embodiment of the smoking substitute system; and
Figure 3 is a perspective view of the device in accordance with an embodiment of the present disclosure;
Figure 4 is cross-sectional view of the device in accordance with an embodiment.

### DETAILED DESCRIPTION OF THE INVENTION

Aspects and embodiments of the present invention will now be discussed with reference to the accompanying figures. Further aspects and embodiments will be apparent to those skilled in the art. All documents mentioned in this text are incorporated herein by reference.

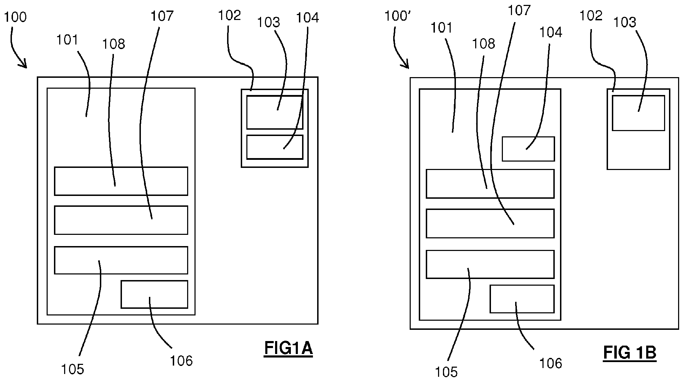

Figure 1A is a schematic providing a general overview of a smoking substitute system 100. The system 100 includes a substitute smoking device 101 and an aerosol-forming article in the form of a consumable 102, which comprises an aerosol former 103. The system is configured to vaporise the aerosol former by heating the aerosol former 103 (so as to form a vapour/aerosol for inhalation by a user).

In the illustrated system, the heater 104 forms part of the device 101 and is configured to heat the aerosol former 103. In this variation, the heater 104 is electrically connectable to the power source 105, for example, when the consumable 102 is engaged with the device 101. Heat from the heater 104 vaporises the aerosol former 103 to produce a vapour. The vapour subsequently condenses to form an aerosol, which is ultimately inhaled by the user.

The system 100 further comprises a power source 105 that forms part of the device 101. In other embodiments the power source 105 may be external to (but connectable to) the device 101. The power source 105 is electrically connectable to the heater 104 such that it is able to supply power to the heater 104 (i.e. for the purpose of heating the aerosol former 103). Thus, control of the electrical connection of the power source 105 to the heater 104 provides control of the state of the heater 104. The power source 105 may be a power store, for example a battery or rechargeable battery (e.g. a lithium ion battery).

The system 100 further comprises an I/O module comprising a connector 106 (e.g. in the form of a USB port, Micro USB port, USB-C port, etc.). The connector 106 is configured for connection to an external source of electrical power, e.g. a mains electrical supply outlet. The connector 106 may be used in substitution for the power source 105. That is the connector 106 may be electrically connectable to the heater 104 so as to supply electricity to the heater 104. In such embodiments, the device may not include a power source, and the power source of the system may instead comprise the connector 106 and an external source of electrical power (to which the connector 106 provides electrical connection).

In some embodiments, the connector 106 may be used to charge and recharge the power source 105 where the power source 105 includes a rechargeable battery.

The system 100 also comprises a user interface (Ul) 107. Although not shown, the UI 107 may include input means to receive commands from a user. The input means of the UI 107 allows the user to control at least one aspect of the operation of the system 100. The input means may, for example, be in the form of a button, touchscreen, switch, microphone, etc.

The UI 107 also comprises output means to convey information to the user. The output means may, for example, comprise lights (e.g. LEDs), a display screen, speaker, vibration generator, etc.

The system 100 further comprises a controller 108 that is configured to control at least one function of the device 101. In the illustrated embodiment, the controller 108 is a component of the device 101, but in other embodiments may be separate from (but connectable to) the device 101. The controller 108 is configured to control the operation of the heater 104 and, for example, may be configured to control the voltage applied from the power source 105 to the heater 104. The controller 108 may be configured to toggle the supply of power to the heater 104 between an on state, in which the full output voltage of the power source 105 is applied to the heater 104, and an off state, in which the no voltage is applied to the heater 104.

Although not shown, the system 100 may also comprise a voltage regulator to regulate the output voltage from the power source 105 to form a regulated voltage. The regulated voltage may then be applied to the heater 104.

In addition to being connected to the heater 104, the controller 108 is operatively connected to the UI 107. Thus, the controller 108 may receive an input signal from the input means of the UI 107. Similarly, the controller 108 may transmit output signals to the UI 107. In response, the output means of the UI 107 may convey information, based on the output signals, to a user. The controller also comprises a memory 109, which is a non-volatile memory. The memory 109 includes instructions, which, when implemented, cause the controller to perform certain tasks or steps of a method.

Figure 1B is a schematic showing a variation of the system 100 of Figure 1A. In the system 100' of Figure 1B, the heater 104 forms part of the device 101, rather than the consumable 102. In this variation, the heater 104 is electrically connected to the power source 105.

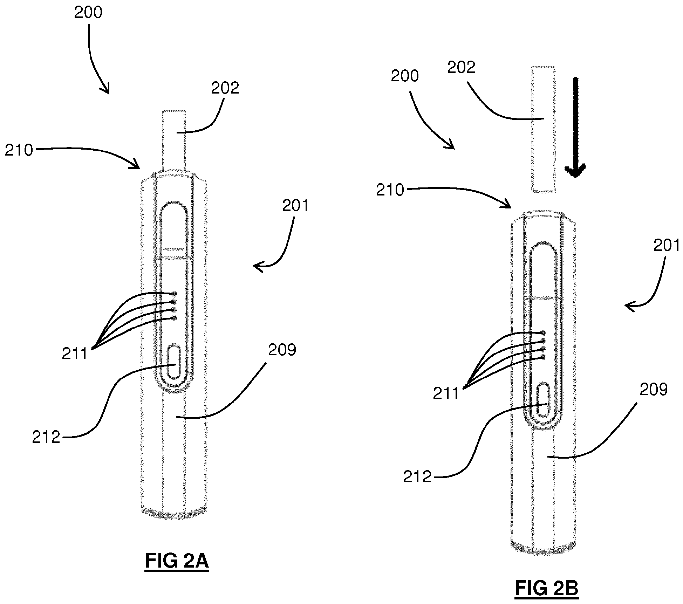

Figures 2A and 2B illustrate a heated-tobacco (HT) smoking substitute system 200. The system 200 is an example of the systems 100, 100' described in relation to Figures 1A or 1B. System 200 includes an HT device 201 and an HT consumable 202. The description of Figures 1A and 1B above is applicable to the system 200 of Figures 2A and 2B, and will thus not be repeated.

The device 201 and the consumable 202 are configured such that the consumable 202 can be engaged with the device 201. Figure 2A shows the device 201 and the consumable 202 in an engaged state, whilst Figure 2B shows the device 201 and the consumable 202 in a disengaged state.

The device 201 comprises a housing 227. The housing 227 defines an external surface 229. The housing 227 may include a body 209 and cap 210. In use the cap 210 is engaged at an end of the body 209. Although not apparent from the figures, the cap 210 is moveable relative to the body 209. In particular, the cap 210 is slideable and can slide along a longitudinal axis of the body 209.

The device 201 comprises an output means (forming part of the UI of the device 201) in the form of a plurality of light-emitting diodes (LEDs) 211 arranged linearly along the longitudinal axis of the device 201 and on an outer surface of the body 209 of the device 201. A button 212 is also arranged on an outer surface of the body 209 of the device 201 and is axially spaced (i.e. along the longitudinal axis) from the plurality of LEDs 211.

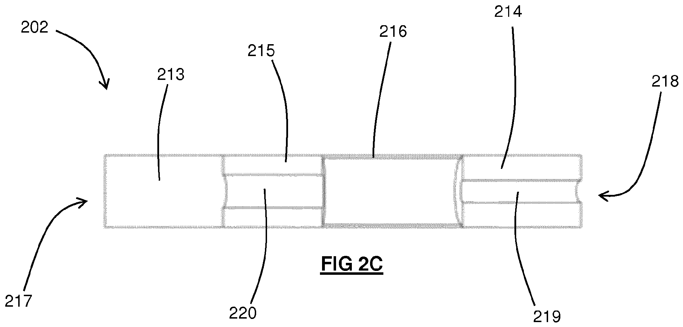

Figure 2C show a detailed section view of the consumable of 202 of the system 200. The consumable 202 generally resembles a cigarette. In that respect, the consumable 202 has a generally cylindrical form with a diameter of 7 mm and an axial length of 70 mm. The consumable 202 comprises an aerosol forming substrate 213, a terminal filter element 214, an upstream filter element 215 and a spacer element 216. In other embodiments, the consumable may further comprise a cooling element. A cooling element may exchange heat with vapour that is formed by the aerosol-forming substrate 213 in order to cool the vapour so as to facilitate condensation of the vapour.

The aerosol-forming substrate 213 is substantially cylindrical and is located at an upstream end 217 of the consumable 202, and comprises the aerosol former of the system 200. In that respect, the aerosol forming substrate 213 is configured to be heated by the device 201 to release a vapour. The released vapour is subsequently entrained in an airflow flowing through the aerosol-forming substrate 213. The airflow is produced by the action of the user drawing on a downstream 218 (i.e. terminal or mouth) end of the consumable 202.

In the present embodiment, the aerosol forming substrate 213 comprises tobacco material that may, for example, include any suitable parts of the tobacco plant (e.g. leaves, stems, roots, bark, seeds and flowers). The tobacco may comprise one or more of leaf tobacco, stem tobacco, tobacco powder, tobacco dust, tobacco derivatives, expanded tobacco, homogenised tobacco, shredded tobacco, extruded tobacco, cut rag tobacco and/or reconstituted tobacco (e.g. slurry recon or paper recon). For example, the aerosol-forming substrate 213 may comprise a gathered sheet of homogenised (e.g. paper/slurry recon) tobacco or gathered shreds/strips formed from such a sheet.

In order to generate an aerosol, the aerosol forming substrate 213 comprises at least one volatile compound that is intended to be vaporised/aerosolised and that may provide the user with a recreational and/or medicinal effect when inhaled. The aerosol-forming substrate 213 may further comprise one or more additives. For example, such additives may be in the form of humectants (e.g. propylene glycol and/or vegetable glycerine), flavourants, fillers, aqueous/non-aqueous solvents and/or binders.

The terminal filter element 214 is also substantially cylindrical, and is located downstream of the aerosol forming substrate 213 at the downstream end 218 of the consumable 202. The terminal filter element 214 is in the form of a hollow bore filter element having a bore 219 (e.g. for airflow) formed therethrough. The diameter of the bore 219 is 2 mm. The terminal filter element 214 is formed of a porous (e.g. monoacetate) filter material. As set forth above, the downstream end 218 of the consumable 202 (i.e. where the terminal filter 214 is located) forms a mouthpiece portion of the consumable 202 upon which the user draws. Airflow is drawn from the upstream end 217, thorough the components of the consumable 202, and out of the downstream end 218. The airflow is driven by the user drawing on the downstream end 218 (i.e. the mouthpiece portion) of the consumable 202.

The upstream filter element 215 is located axially adjacent to the aerosol-forming substrate 213, between the aerosol-forming substrate 213 and the terminal filter element 214. Like the terminal filter 214, the upstream filter element 215 is in the form of a hollow bore filter element, such that it has a bore 220 extending axially therethrough. In this way, the upstream filter 215 may act as an airflow restrictor. The upstream filter element 215 is formed of a porous (e.g. monoacetate) filter material. The bore 220 of the upstream filter element 215 has a larger diameter (3 mm) than the terminal filter element 214.

The spacer 216 is in the form of a cardboard tube, which defines a cavity or chamber between the upstream filter element 215 and the terminal filter element 214. The spacer 216 acts to allow both cooling and mixing of the vapour/aerosol from the aerosol-forming substrate 213. The spacer has an external diameter of 7 mm and an axial length of 14mm.

Although not apparent from the figure, the aerosol-forming substrate 213, upstream filter 215 and spacer 216 are circumscribed by a paper wrapping layer. The terminal filter 214 is circumscribed by a tipping layer that also circumscribes a portion of the paper wrapping layer (so as to connect the terminal filter 214 to the remaining components of the consumable 202). The upstream filter 215 and terminal filter 214 are circumscribed by further wrapping layers in the form of plug wraps.

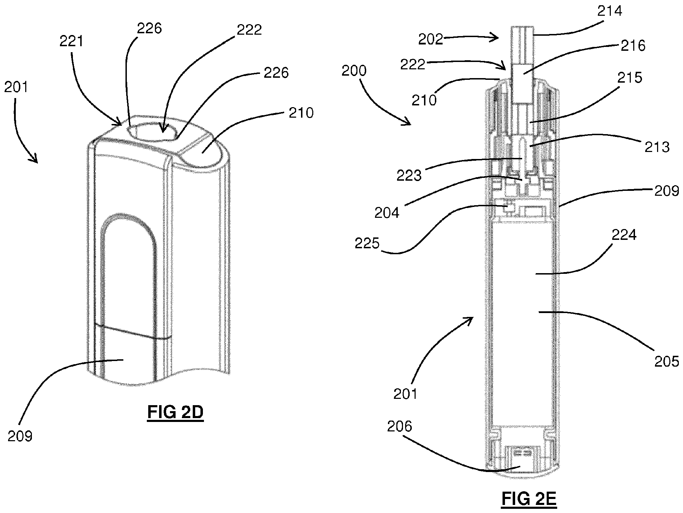

Returning now to the device 201, Figure 2D illustrates a detailed view of the end of the device 201 that is configured to engage with the consumable 202. The cap 210 of the device 201 includes an opening 221 to an internal cavity 222 (more apparent from Figure 2D) defined by the cap 210. The opening 221 and the cavity 222 are formed so as to receive at least a portion of the consumable 202. During engagement of the consumable 202 with the device 201, a portion of the consumable 202 is received through the opening 221 and into the cavity 222. After engagement (see Figure 2B), the downstream end 218 of the consumable 202 protrudes from the opening 221 and thus also protrudes from the device 201. The opening 221 includes laterally disposed notches 226. When a consumable 202 is received in the opening 221, these notches 226 remain open and could, for example, be used for retaining a cover in order to cover the end of the device 201.

Figure 2E shows a cross section through a central longitudinal plane through the device 201. The device 201 is shown with the consumable 202 engaged therewith.

The device 201 comprises a heater 204 comprising heating element 223. The heater 204 forms part of the body 209 of the device 201 and is rigidly mounted to the body 209. In the illustrated embodiment, the heater 204 is a rod heater with a heating element 223 having a circular transverse profile. In other embodiments the heater may be in the form of a blade heater (e.g. heating element with a rectangular transverse profile) or a tube heater (e.g. heating element with a tubular form).

The heating element 223 of the heater 204 projects from an internal base of the cavity 222 along a longitudinal axis towards the opening 221. As is apparent from the figure, the length (i.e. along the longitudinal axis) of the heating element is less than a depth of the cavity 222. In this way, the heating element 223 does not protrude from or extend beyond the opening 221.

When the consumable 202 is received in the cavity 222 (as is shown in Figure 2E), the heating element 223 penetrates the aerosol-forming substrate 213 of the consumable 202. In particular, the heating element 223 extends for nearly the entire axial length of the aerosol-forming substrate 213 when inserted therein. Thus, when the heater 204 is activated, heat is transferred radially from an outer circumferential surface of the heating element 223 to the aerosol-forming substrate 213.

The device 201 further comprises an electronics cavity 224. A power source, in the form of a rechargeable battery 205 (a lithium ion battery), is located in electronics cavity 224.

The device 201 includes a connector (i.e. forming part of an IO module of the device 201) in the form of a USB port 206. The connector may alternatively be, for example, a micro-USB port or a USB-C port for examples. The USB port 206 may be used to recharge the rechargeable battery 205.

The device 201 includes a controller (not shown) located in the electronics cavity 224. The controller comprises a microcontroller mounted on a printed circuit board (PCB). The USB port 206 is also connected to the controller 208 (i.e. connected to the PCB and microcontroller).

The controller 208 is configured to control at least one function of the device 202. For example, the controller 208 is configured to control the operation of the heater 204. Such control of the operation of the heater 204 may be accomplished by the controller toggling the electrical connection of the rechargeable battery 205 to the heater 204. For example, the controller 208 is configured to control the heater 204 in response to a user depressing the button 212. Depressing the button 212 may cause the controller to allow a voltage (from the rechargeable battery 205) to be applied to the heater 204 (so as to cause the heating element 223 to be heated).

The controller is also configured to control the LEDs 211 in response to (e.g. a detected) a condition of the device 201 or the consumable 202. For example, the controller may control the LEDs to indicate whether the device 201 is in an on state or an off state (e.g. one or more of the LEDs may be illuminated by the controller when the device is in an on state).

The device 201 comprises a further input means (i.e. in addition to the button 212) in the form of a puff sensor 225. The puff sensor 225 is configured to detect a user drawing (i.e. inhaling) at the downstream end 218 of the consumable 202. The puff sensor 225 may, for example, be in the form of a pressure sensor, flowmeter or a microphone. The puff sensor 225 is operatively connected to the controller 208 in the electronics cavity 224, such that a signal from the puff sensor 225, indicative of a puff state (i.e. drawing or not drawing), forms an input to the controller 208 (and can thus be responded to by the controller 208).

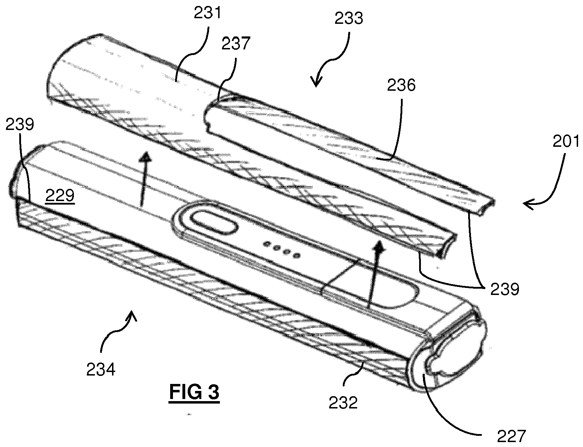

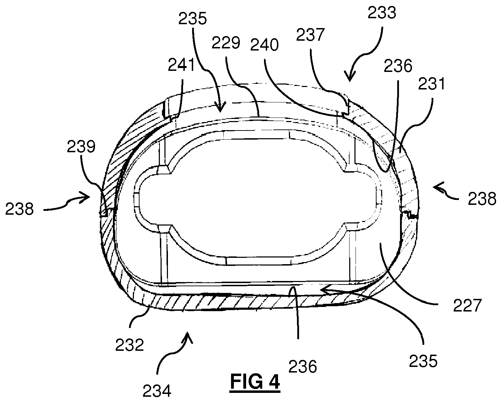

Figure 3 and Figure 4 illustrate the device 201 in accordance with an embodiment. The device 201 comprises the housing 227 and at least one panel 231, 232. The housing 227 is configured for housing components of the device such as, the heater 204, the power source 105, the controller 208, the puff sensor 225, and like. The housing 227 defines an external surface 229. The user interface 107 is positioned on the external surface 229 of the housing 227. For example, in the embodiment as shown, the button 212 and the LEDs 211 are arranged on the external surface 229.

The panels 231, 232 are configured for covering at least a portion of the external surface 229 of the housing 227. Further, as shown in Figure 4, the panels 231, 232 are further configured to space from the external surface 229 to define a gap 235 between an internal surface of the panel and the external surface 229 of the housing 227. The gap 235 defines a void and thereby it is filled with air. The panels 231, 232 have shape and configuration as suitable for the shape and size of the device. In the embodiment as illustrated, the panel includes a first panel 231 configured for placement over the external surface 229 at a front portion 233 of the housing 227 and a second panel 232 configured for placement over the external surface 229 on a back portion 234 of the housing 227. Each of the first panel 231 and the second panel 232 at least partly space from the external surface 229 to form the gap 235 between an external surface 229 of the housing and an internal surface 236 of the panel.

The panels 231, 232 are configured as required based on the configuration of the housing 227. In the embodiment as shown, the first panel 231 is provided with a cut-out 237 corresponding to the shape and position of the UI of the device 201. The cut-out 237 makes the UI accessible to a user. In an embodiment, instead of or in addition to the cut-out 237, a transparent covering may be provided such that UI may be accessible to a user.

The gap 235 is configured based on insulation requirement. For example, the gap 235 may be provided either over substantially entire length of the housing 227 or may be localized based on location of the heat generation elements in the housing 227. In the embodiment as shown, the gap 235 is wider at the front portion 233 and the back portion 234 that that is provided at the side portions 238 of the device.

Further, the panels 231, 232 are removably attached with the device 201 using a suitable means. In the illustrated embodiment, the panel is configured for a snap engagement with the device 201. The snap engagement provides for a continent mounting and/or unmounting of the panel from the device 201. In addition, the panels 231, 232 are provided with different designs, colours, finishes or material. This may enable users to customize the appearance and feel of the device 201 according to their personal preferences. For example, the panels 231, 232 are provided with a non-scratch surface finish to prevent formation of any scratched on the panel. In an embodiment, the panels 231, 232 are provided with an elastic coating of a deformable material such as rubber or silicon to enhance grip of the device or to improve shock resistance of the device 201.

In the embodiment as shown, the first panel 231 is configured for snap engagement with the second panel 232 for retainment of the panel over the external surface 229. Longitudinal edges 239 of the first panel 231 and the second panel 232 are configured for removable attachment with each other. A longitudinal edge of the first panel 231 snap locks with the longitudinal edge of the second panel 232 for mounting the panel over and covering the external surface 229 of the housing 227. Further, the first panel 231 is provided with a rib 240 defining the periphery of the cut-out 237. The rib 240 is configured for positioning inside a groove 241 provided in the housing 227 surrounding the UI. For mounting the first panel 231 on the housing 227, the first panel 231 may either be pushed into a snap lock engagement of the rib 240 in the groove 241, or the first panel 231 may be configured to be slid along a longitudinal axis of the housing 227 for inserting the rib 240 in the groove 241. Any suitable provision may be provided as required.

In some embodiments, the panels 231, 232 may be provided with at least one perforation. The perforations may provide for passage of air between the gap 235 and the environment. The passage of air may provide for a vent for heated air from the gap 235 and for receiving cool air from the environment. This aid in cooling of the device 201 and also prevents any pressure build up in the gap 235 due to repeated heating/cooling of the device. The perforations may be in form of holes in the panel or may be in form of crevices between the housing 227 and the panel.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the words "have", "comprise", and "include", and variations such as "having", "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means, for example, +/- 10%.

The words "preferred" and "preferably" are used herein refer to embodiments of the invention that may provide certain benefits under some circumstances. It is to be appreciated, however, that other embodiments may also be preferred under the same or different circumstances. The recitation of one or more preferred embodiments therefore does not mean or imply that other embodiments are not useful, and is not intended to exclude other embodiments from the scope of the disclosure, or from the scope of the claims.

## Claims

1. A smoking substitute device comprising:
a housing configured to house components of the smoking substitute device; and
a panel configured to cover an external surface of the housing;
wherein the panel is configured to space from said external surface so as to provide thermal insulation to the housing.

2. A smoking substitute device according to claim 1, wherein the panel is removably attachable to the housing.

3. A smoking substitute device according to any one of the preceding claims, wherein the panel is configured to allow access to a user interface positioned on the external surface.

4. A smoking substitute device according to any one of the preceding claims, wherein the panel comprises a window adjacent to a visual indicator provided on the housing.

5. A smoking substitute device according to any one of the preceding claims, wherein the panel comprises a first panel configured to cover a first external surface at the front of the device and a second panel configured to cover a second external surface at the back of the device opposite to the front of the device.

6. A smoking substitute device according to claim 5, wherein the first panel is configured to engage with the second panel when the first panel and the second panel to cover the respective external surface of the housing.

7. A smoking substitute device according to claim 5 or claim 6, wherein the first panel is configured to form a snap lock engagement with the second panel.

8. A smoking substitute device according to any of the preceding claims, wherein the panel is configured to cover the external surface by attaching to the housing with one or more of a magnetic engagement, a living hinge engagement, a snap groove engagement, or runner formed between the panel and the housing.

9. A smoking substitute device accordingly to any preceding claim, wherein one or more vents are defined between the external surface and the panel.

10. A smoking substitute device accordingly to any preceding claim, wherein the panel comprises a non-scratch surface finish.

11. A smoking substitute device accordingly to any preceding claim, wherein the panel comprises an elastic coating.

12. A smoking substitute device accordingly to any preceding claim, wherein a portion of the panel comprises a planar surface corresponding to the external surface of the device.

13. A smoking substitute device accordingly to any preceding claim, wherein the smoking substitute device is a heat-not-burn (HNB) device.

14. A smoking substitute system, comprising:
i) a smoking substitute device according to any one of claims 1 to 13; and
ii) an aerosol-forming article.

15. A smoking substitute system of claim 14, wherein the aerosol-forming article comprises heated tobacco (HT) consumable.
